Europäisches Patentamt

European Patent Office

Office européen des brevets

(19)

(11) EP 0 752 249 A2

(12) # DEMANDE DE BREVET EUROPEEN

(43) Date de publication:
08.01.1997 Bulletin 1997/02

(51) Int Cl.6: **A61K 45/06**, A61K 31/415
// (A61K31/415, 31:34)

(21) Numéro de dépôt: 96401284.3

(22) Date de dépôt: 13.06.1996

(84) Etats contractants désignés:
AT BE CH DE DK ES FI FR GB GR IE IT LI LU MC NL PT SE

(30) Priorité: 14.06.1995 FR 9507064

(71) Demandeur: SANOFI
75374 Paris Cédex 08 (FR)

(72) Inventeur: **Nisato, Dino**
**34680 Saint Georges d'Orques (FR)**

(74) Mandataire: **Gillard, Marie-Louise et al**
**Cabinet Beau de Loménie**
**158, rue de l'Université**
**75340 Paris Cédex 07 (FR)**

(54) **Utilisation d'un antagoniste de l'angiotensine II et d'un dérivé du benzofurane à activité antiarythmique pour la préparation d'un médicament utile dans le traitement des affections cardiovasculaires**

(57) L'invention concerne l'utilisation d'un antagoniste de l'angiotensine II et d'un dérivé du benzofurane à activité antiarythmique pour la préparation d'une composition pharmaceutique utile dans le traitement des affections cardiovasculaires, notamment dans le traitement de la défaillance cardiaque.

EP 0 752 249 A2

**Description**

La présente invention a pour objet l'utilisation conjointe d'un antagoniste de l'angiotensine II et d'un dérivé du benzofurane, pour le traitement des affections cardiovasculaires, en particulier le traitement de la défaillance cardiaque.

Plus particulièrement la présente invention concerne l'utilisation de l'irbesartan et de l'amiodarone, dans le traitement des affections cardiovasculaires, notamment dans le traitement de la défaillance cardiaque.

La présente invention a également pour objet une nouvelle composition pharmaceutique utile en particulier pour le traitement de la défaillance cardiaque. Cette composition pharmaceutique est constituée de 2 principes actifs, l'un étant un dérivé du benzofurane, connu pour son activité comme antiarythmique, l'autre étant un composé antagoniste de l'angiotensine II.

La défaillance cardiaque est définie comme un état pathologique lié à une diminution de la contractilité du myocarde et dans lequel le coeur est incapable de pomper la quantité de sang nécessaire pour satisfaire la demande métabolique de différents organes et tissus de l'organisme.

D'après l'étude de Framingham (P.A. McKee et al., New Engl. J. Med. 1971, 285, 1441-1446) il apparait que la défaillance cardiaque n'est pas une maladie en soi mais une manifestation clinique de différents troubles cardiaques. En fait plusieurs formes de pathologies cardiaques peuvent conduire à un dysfonctionnement ventriculaire et causer le syndrome de défaillance cardiaque. Ainsi la défaillance cardiaque étant une somme de syndromes évoluant différemment et présentant diverses étiologies, il est clair qu'un seul agent thérapeutique ne peut suffire à son traitement.

Il y a une relation importante entre l'hypertrophie ventriculaire gauche et la mort subite, relation expliquée en partie par l'association de l'hypertrophie ventriculaire gauche et de l'arythmie ventriculaire.

Les patients atteints de défaillance cardiaque sévère présentent un taux élevé d'activité ventriculaire ectopique et la mort subite due aux arythmies cardiaques est la cause de plus de 40 % des morts par défaillance cardiaque.

Le taux élevé de mortalité chez les patients atteints de défaillance cardiaque a conduit à rechercher des nouvelles thérapeutiques permettant de prolonger leur vie.

L'utilisation de vasodilatateurs semble justifiée pour le traitement de la défaillance cardiaque. De plus on obtient une amélioration de l'hémodynamique et du profil neuroendocrinien par un traitement prolongé avec des digitaliques, des glycosides, et/ou des diurétiques et des vasodilatateurs.

On peut également attendre une amélioration dans le traitement de la défaillance cardiaque en utilisant un inhibiteur de l'enzyme de conversion seul ou en association avec d'autres agents thérapeutiques.

Les résultats de l'étude CONSENSUS I (N. Engl. J. Med., 1992, 327, 678-684) de l'étude SAVE (N. Engl. J. Med., 1992, 327, 669-677) et SOLVD (N. Engl. J. Med., 1991, 325, 293-302) indiquent clairement que les inhibiteurs de l'enzyme de conversion peuvent être utilisés pour traiter des patients souffrant de défaillance cardiaque faible, modérée ou grave associée à des dysfonctionnements systoliques ventriculaires gauches. Dans ce cas les inhibiteurs de l'enzyme de conversion améliorent la qualité de la vie et la survie des patients.

Actuellement, quelle que soit l'origine de la défaillance cardiaque, les patients sont traités dans la majorité des cas par une combinaison de plusieurs médicaments : des digitaliques, des diurétiques et inhibiteurs de l'enzyme de conversion (J.G.F. Cleland et al., Br. Heart J., 1994, 72 (2, suppl.) 573-579).

La demande de brevet internationale WO 93-20839 décrit l'utilisation à long terme d'un inhibiteur du système rénine-angiotensine tel qu'un inhibiteur de l'enzyme de conversion, un inhibiteur de la rénine ou un antagoniste de l'angiotensine II pour réduire la mortalité après un infarctus du myocarde. D'après cette invention l'inhibiteur du système rénine-angiotensine peut être administré en association avec un autre principe actif tel qu'un agent bloquant béta-adrénergique, un anticoagulant ou l'aspirine.

La demande de brevet canadien 2 070 085 décrit une composition pharmaceutique à libération prolongée utile pour le traitement de l'hypertension, de la défaillance cardiaque et d'autres problèmes coronaires comprenant un agent bloquant des canaux calciques et un inhibiteur de l'enzyme de conversion.

L'action bénéfique du losartan, un inhibiteur de l'angiotensine II, sur les patients souffrant de défaillance cardiaque est montrée dans J. Hypertension, 1994, 12 (suppl. 2), p. S31-S35.

Le brevet américain 4 868 179 revendique une méthode pour diminuer la mortalité associée à la défaillance cardiaque chronique comprenant l'administration orale d'une composition pharmaceutique comprenant de l'hydralazine et du dinitrate d'isosorbide.

Le brevet européen 0 527 720 décrit une méthode de traitement de la défaillance cardiaque par administration d'une composition pharmaceutique comprenant un inhibiteur de l'enzyme de conversion et la 7-fluoro-1-méthyl-3-méthylsulfinyl-4-quinolone.

La demande de brevet internationale WO 90/09171 décrit des préparations médicamenteuses cardio-protectrices utiles dans l'insuffisance coronaire, la prévention de la survenue d'un infarctus ou l'apparition de la mort subite, comprenant l'amiodarone, un dérivé nitré et facultativement un béta-bloquant.

Par dérivé du benzofurane à activité antiarythmique, on entend un composé tel que décrit dans un des documents ci-après : brevets US 3 248 401 et 5 223 510, brevet européen 338 746, demandes de brevet WO 88/07996, WO 89/02892, WO 90/2743, WO 94/29289.

Parmi ces composés, on préfère l'amiodarone décrite dans le brevet US 3 248 401 et la N,N-dibutyl-3-[4-((2-butyl-5-méthylsulfonamido)benzofuran-3-yl-carbonyl)phénoxy]propylamine ou dronédarone, dénommée également SR 33589 et ses sels décrits dans le brevet US 5 223 510.

Les métabolites actifs de ces composés sont également des composés préférés notamment la N-deséthylamiodarone et ses sels décrits dans le brevet français 2 550 091 et la N-butyl-3-[4-((2-butyl-5-méthylsulfonamido)benzofuran-3-ylcarbonyl) phenoxy]propylamine et ses sels décrits dans le brevet US 5 223 510.

L'amiodarone est considérée comme un antiarythmique de classe III (B.N. Singh et al., Current Opinion in Cardiology, 1994, 9, 12-22). Elle est utilisée mondialement pour traiter les arythmies ventriculaires et supraventriculaires.

De plus, des études préliminaires relatives à l'utilisation prophylactique de l'amiodarone chez des patients souffrant de défaillance cardiaque, de tachycardie ventriculaire non soutenue ou de ces 2 syndromes donnent des résultats prometteurs.

Ainsi, l'étude GESICA (H.C. Doval et al., Lancet, 1994, 344, 493-498) décrit des observations faites sur des patients souffrant de défaillance cardiaque grave qui ont été suivis pendant 2 ans. Cette étude montre que le traitement par l'amiodarone à faible dose diminue la mortalité et les hospitalisations dans tous les sous-groupes examinés et indépendamment d'une éventuelle tachycardie ventriculaire non soutenue.

Une autre étude (J.J. Mahmaria et al., Am. J. Cardiol. 1994, 74, 681-686) conduite sur des patients présentant une fraction d'éjection ventriculaire gauche inférieure à 40 %, montre que l'amiodarone administrée à des doses journalières de 50 à 100 mg améliore l'hémodynamique cardiaque et la contractilité.

Chez des patients présentant une arythmie sévère secondaire à une cardiomyopathie congestive, l'amiodarone constitue un traitement de choix, en association avec les inhibiteurs de l'enzyme de conversion, les digitaliques ou les diurétiques (Acta Med. Austriaca, 1992, 19 (3), 83-87).

La demande de brevet WO 95/09625 décrit l'utilisation de l'amiodarone dans le traitement de la défaillance cardiaque, dans ce cas l'amiodarone peut être associée à un autre agent thérapeutique tel que, par exemple, un diurétique, un cardiotonique, un vasodilatateur ou un inhibiteur de l'enzyme de conversion.

Ainsi l'association d'un antagoniste de l'angiotensine II à l'amiodarone n'a jamais été envisagée, à ce jour, pour le traitement des affections cardiovasculaires.

Selon la présente invention, par antagoniste de l'angiotensine II, on entend des composés non peptidiques qui présentent une forte affinité pour les récepteurs de l'angiotensine II du sous-type AT1 : (M.I. Steinberg *et al.,* Cardiovascular Drug Reviews, 1993, 11(3), 312-358). Il s'agit généralement d'hétérocycles azotés substitués par un groupe biphénylméthyle portant lui-même un groupe acide. Parmi les hétérocycles azotés on peut citer en particulier les imidazoles ainsi que d'autres cycles à 5 chaînons tels que des pyrroles, pyrazoles, isoxazoles, isothiazoles et triazoles. De tels composés sont décrits dans les brevets ou demandes de brevet suivants : EP 28 834-A, EP 253 310-A, EP 324 377-A, WO 91/14679, EP 392 317-A, EP 403 159-A, EP 573 271-A, WO 94/08989, WO 94/08990, US 4 576 958, US 4 582 847, US 4 207 324, US 4 340 598.

D'autres composés inhibiteurs de l'angiotensine II sont décrits dans des brevets ou demandes de brevets relatifs à des dérivés formés sur des hétérocycles condensés notamment des benzimidazoles et des imidazopyridines : EP 399 731-A, EP 400 974-A, EP 392 317-A, EP 260 613-A, EP 412 848-A, EP 420 237-A, EP 502 314-A, EP 503 162-A, EP 552 765-A, EP 546 358-A, EP 595 151-A, EP 598 702-A, EP 245 637-A, US 4 880 804, WO 93/190067, WO 94/01436, WO 94/204 498.

Par ailleurs, d'autres composés inhibiteurs de l'angiotensine II sont formés à partir d'hétérocycles azotés à 6 chaînons éventuellement condensés. De tels composés sont décrits en particulier dans les brevets ou demandes de brevets suivants : EP 412 848-A, GB 2 234 748-A, WO 91/07404, EP 487 252-A, EP 443 983-A, EP 403 159-A, EP 434 249-A, EP 500 409-A, WO 94/03449, WO 94/07492, WO 94/11379, WO 94/11369, WO 95/002596.

Enfin d'autres types de structure ont été décrits pour des composés inhibiteurs de l'angiotensine II, notamment dans les brevets ou demandes de brevet suivants : EP 566 060-A, WO 94/00450, EP 604 259.

De façon particulière, on peut citer les composés suivants parmi les antagonistes de l'angiotensine II convenant pour leur utilisation selon l'invention et dans les compositions pharmaceutiques selon l'invention ; ces composés sont connus par leur dénomination commune internationale ou par leur nom de code, la structure chimique associée à chaque code étant indiquée ci-après ou décrite dans les Chemical Abstracts:

Irbesartan, losartan, valsartan, zolasartan, telmisartan,

A-81282 : Acide 4-(N-butyl-N-[(2'-[1H-tétrazol-5-yl] biphényl-4-yl)méthyl] amino)pyrimidine-5-carboxylique,

A-81988 : Acide 2-[N-propyl-N-[2'-(1H-tétrazol-5-yl)biphényl-4-ylméthyl] amino]pyridine-3-carboxylique,

ANA-756 ou tasosartan,

BMS-184 698 : Ester méthylique de l'acide 2-cyclopropyl-3-[(2'-(1H-tétrazol-5-yl)[1,1'-biphényl]-4-yl] oxy]-, (5-méthyl-2-oxo-1,3-dioxol-4-yl)quinoline carboxylique,

CGP-49870 : Acide 1-[[N-(1-oxopentyl)-N-[2'-(1H-tétrazol-5-yl)[[1,1'-biphényl]-4-yl]méthyl]amino]méthyl]cyclopentanecarboxylique,

CI-996 : Acide 2-propyl-4-[(3-trifluoroacétyl)pyrrol-1-yl]-1-[[2'-(1H-tétrazol-5-yl) [1,1-biphényl]-4-yl] méthyl]-1H-imidazole-5-carboxylique,

CL-329167 : 2-Butyl-6-(1-méthoxy-1-méthyléthyl)-3-[[2'-(1H-tétrazol-5-yl)[1,1'-biphényl]-4-yl]méthyl]-4(3H)-quinazolinone,

CP-161418 : Acide 2-butyl-4-chloro-1-(5-(2-(1H-tétrazol-5-yl)phényl)pyrimidin-2-ylméthyl)imidazole-5-carboxylique,

D-8731 : 2-Ethyl-4-[[2'-(1H-tétrazol-5-yl)[1,1'-biphényl]-4-yl]méthoxy] quinoline,

DMP-581 : 4-Ethyl-2-propyl-1-[[2'-1H-tetrazol-5-yl)[1,1'-biphényl]-4-yl] méthyl]-1H-imidazole-5-carboxaldéhyde,

DMP-811 : Acide 4-éthyl-2-propyl-1-[[2'-(1H-tétrazol-5-yl)[1,1'-biphényl]-4-yl]méthyl]1H-imidazole-5-carboxylique,

DUP-532 Acide 4-(pentafluoroethyl)-2-propyl-1-[[2'-(1H-tetrazol-5-yl)[1,1'-biphényl]-4-yl]methyl]-1H-imidazole-5-carboxylique,

E-4177 : Acide 4'-[(2-cyclopropyl-7-méthyl-3H-imidazo[4,5-b]pyridin-3-yl)méthyl]-[1,1'-biphenyl]-2-carboxylique,

EMD-66397 : Sel de potassium de l'esther éthylique de l'acide 2-(2-butyl-4,5-dihydro-4-oxo-3-(2'-(1H-tétrazol-5-yl)-4-biphénylméthyl)-3H-imidazo(4,5-c)pyridine-5-ylméthyl)-benzoïque,

EXP-408 : Ester méthylique de l'acide 4-éthyl-1-(3-fluoro-2'-(3-méthylbutoxycarbonylaminosulfonyl)biphényl-4-ylméthyl)-2-propylimidazole-5-carboxylique,

EXP-970,

EXP-3174 : Acide 2-butyl-4-chloro-1-[(2'-(1H-tétrazol-5-yl)(1,1'-biphényl)-4-yl)méthyl-1H-imidazole-5-carboxylique,

EXP-3892 : Acide 2-propyl-1-[[2'-(1H-tétrazol-5-yl)[1,1'-biphényl]-4-yl]méthyl]-4-(trifluorométhyl)-1H-imidazole-5-carboxylique,

EXP-6803 : 2-[4-[2-n-Butyl-4-chloro-5-(méthoxycarbonylméthyl)-1-imidazolyl méthyl]phénylaminocarbonyl]-benzoate de sodium,

EXP-7711 : Acide 4'-[2-butyl-4-chloro-5-(hydroxyméthyl)imidazol-1-yl-méthyl]-1,1'-biphényl-2-carboxylique,

GA-0050 : 2-(6-((2-Ethyl-5,7-diméthyl-3H-imidazo(4,5-b)pyridin-3-yl)méthyl)quinolin-2-yl)benzoate de sodium,

GR-138 950 : 1-((3-bromo-2-(2-(((trifluorométhyl)sulfonyl)amino)phényl)-5-benzofuranyl)méthyl)-4-cyclopropyl-2-éthyl-1H-imidazole-5-carboxamide,

HN-65 021 : ester 1-[(éthoxycarbonyl)oxy]éthyl de l'acide 2-butyl-4-chloro-1-[[2'-(1H-tétrazol-5-yl)[1,1'-biphényl]-4-yl]méthyl]-1H-imidazole-5-carboxylique,

KT-3671 : 5,6,7,8-Tétrahydro-2-propyl-3-[[2'-(1H-tétrazol-5-yl)[1,1'-biphényl]-4-yl]méthyl]-4(3H)-cycloheptimidazolone,

KW-3433 : 2-(2-Ethyl-5,7-diméthyl-3H-imidazo(4,5-b)pyridin-3-ylméthyl)-5-(2H-tétrazol-5-ylméthyl)-10,11-dihydro-5H-dibenz(b,f)azepine,

L-158 809 : 5,7-Diméthyl-2-éthyl-3-((2'-(1H-tétrazol-5-yl)(1,1'-biphényl)-4-yl) méthyl)-3H-imidazo-(4,5)pyridine monohydrate,

L-158 978 : Acide 2-éthyl-7-méthyl-3-[2'-(tétrazol-5-yl)biphényl-4-yl-méthyl]-3H-imidazol [4,5-b]pyridine-5-carboxylique,

L-159 282,

L-159 686 : Acide 1,4-bis(N,N-diphénylcarbamoyl)pipérazine-2(S)-carboxylique.

L-159 689 : N-[3,4-dihydro-4-oxo-2-propyl-3-[2'-(1H-tétrazol-5-yl)[1,1'-biphényl]-4-yl]méthyl]-6-quinazolinyl]-N-pentyl-benzamide,

L-159 874 : Acide 1-(N,N-bis(3-chlorophényl)carbamoyl)-4-(N,N-dipentylcarbamoyl)pipérazine-2(S)-carboxylique,

L-161 177 : 2-Ethyl-5,7-diméthyl-3-[[2'-(3H-1,2,3,5-oxathiadiazol-4-yl)[1,1'-biphényl]-4-yl]méthyl]-, S-oxide-3H-imidazo[4,5-b]pyridine,

L-161 816 : Acide 3-(3,5-dibromo-4-hydroxybenzyl)-7-méthyl-2-propyl-3H-imidazo[4,5-b]pipéridine-5-carboxylique méthyl ester, sel de potassium,

L-162 154,

L-162 234 : 1,1-Diméthyléthyl-2-(4'-(1-(3-(5-butyl)-2-oxo-2(2-triflylphényl)-(1,3,4-triazolyl)méthyl)biphényl)sulfonylaminocarboxylate,

L-162 441 : Ester butylique de l'acide [[4'-[[2-butyl-5-méthyl-6-[(1-oxopentyl)amino]-3H-imidazo [4,5-b]pyridin-3-yl]méthyl][1,1'-biphényl]-2-yl] sulfonyl]-carbamique,

L-163 007 :acide N-(4'-(3-Butyl-5-oxo-1-(5-propionamido-2-(trifluorométhyl)-phenyl)-4,5-dihydro-1H-1,2,4-triazol-4-ylméthyl)biphényl-2-yl -sulfonyl) carbamique.

L-163 017 : Ester butylique de l'acide [[4'-[[6-(benzoylamino)-7-méthyl-2-propyl-3H-imidazo[[4'5-b]pyridin-3-yl]méthyl][1,1'-biphényl]-2-yl]sulfonyl]-, 3-méthylcarbamique,

LF-7 -0156 : Acide 2-[[[2-butyl-1-[(4-carboxyphényl)méthyl]-1H-imidazol-5-yl]méthyl]amino] benzoïque,

LR-B-081 : Méthyl 2-((4-butyl-3-méthyl-6oxo-((2'-(1H-tétrazol-5-yl)-(1,1'-biphényl)-4-yl)méthyl)-1-(6H)-pyrimidinyl)méthyl)-3-thiophéncarboxylate,

LY-285 434 : Acide 2-(5-(2-(2H-tétrazol-5-yl)phényl)-1H-indol-1-yl)octanoïque,

ME-3221 : 3-Méthoxy-2,6-diméthyl-4-[[2'-(1H-tétrazol-5-yl)[1'-biphényl]-4-yl]méthoxy]-pyridine,

MK-996 : N-[[4'-[(2-Ethyl-5,7-diméthyl-3H-imidazo[4,5-b]pyridin-3-yl)méthyl][1,1'-biphényl]-2-yl]sulfonyl]-benzamide,

PD-123 177 : Acide 1-[(4-amino-3-méthylphényl)méthyl]-5-(diphénylacétyl)-4,5,6,7-tétrahydro-1H-imidazo[4,5-c]pyridine-6-carboxylique,

PD-123 319 : Acide 1-[4-(diméthylamino)-3-méthylbenzyl]-5-(diphénylacétyl)-4,5,6,7-tétrahydro-1H-imidazo[4,5-c]pyridine-6(S)-carboxylique,

PD-150 304 : Acide 4-(2-butyl-5-(1-butyl-3-(5-méthylthiophen-3-ylméthyl)-2,5-dioxoimidazolidin-4-ylidèneméthyl)imidazol-1-ylméthyl)benzoïque, RWJ-38970,

RWJ 46458 : Ester éthylique de l'acide [4-éthyl-4-méthyl-6-oxo-1-[[2'-(1H-tétrazol-5-yl)[1,1'-biphényl]-4-yl]méthyl]-2-pipéridinylidène]-acétique,

SC-48742 : Acide [1,1'-biphényl]-2-carboxylique, 4'-[[2-butyl-4-chloro-5-(hydroxyméthyl)-1H-imidazol-1-yl]méthyl],

SC-50560 : 5-[4'-(3,5-Dibutyl-1,2,4-triazol-1-ylméthyl)biphényl-2-yl]-1H-tétrazole,

SC-51316 : 2,5-Dibutyl-4-[2'-(1H-tétrazol-5-yl)biphényl-4-ylméthyl]-3,4-dihydro-2H-1,2,4-triazol-3-one,

SC-51895 : 1,4-Dibutyl-3-[2'-(1H-tétrazol-5-yl)biphényl-4-ylméthyl]-2,3-dihydro-1H-imidazolin-2-one,

SC-52458 ou forasartan : 5-[(3,5-dibutyl-1H-1,2,4-triazol-1-yl)méthyl]-2-[2-(1H-tétrazol-5-yl)phényl]-pyridine,

SKF-108 566 ou éprosartan,

SL-910 102 : 6-Butyl-(2-phényléthyl)-5-((2'-(1H-tétrazol-5-yl)(1,1'-biphényl)-4-yl)méthyl-4(1H)pyrimidinone,

TAK-536 : Acide 2-éthoxy-1-((2'-(5-oxo-2,5-dihydro-1,2,4-oxadiazol-3-yl)-biphényl-4-yl)méthyl)1H-benzimidazole-7-carboxylique,

TCV-116 ou candesartan,

U-96 849 : 5,8-Ethano-5,8-diméthyl-2-n-propyl-5,6,7,8-tétrahydro-1-[[2'-(1H-tétrazol-5-yl)(1,1'-biphényl)-4-yl]méthyl]-1H,4H-1,3,4a,8a,tétraazacyclo pentanaphtalène-9-one,

UP-269-6 : 5-Méthyl-7-propyl-8-((2'-(1H-tétrazol-5-yl)biphényl-4-yl)méthyl)-1,2,4-triazolo(1,5-c)pyrimidin-2(3H)-one,

UP-27 522 : 7-Butyl-5-hydroxy-6-(2'-(1H-tétrazol-5-yl)biphényl-4-yl-méthyl)-1,2,4-triazolo(1,5-a)pyrimidine,

UR-7198,

WAY-126 227 : 5,6,7,8-Tétrahydro-N-[2'-(1H-tétrazol-5-yl)[1,1'-biphényl]-4-yl]méthyl]-2-(trifluorométhyl)-4-quinazolinamine,

WK-1492 : Sel dipotassique de l'acide 2-(5-éthyl-3-(2-(1H-tétrazol-5-yl)biphényl-4-yl)méthyl-1,3,4-thiazoline-2-ylidène)aminocarbonyl-1-cyclopenten carboxylique,

XH-148 : Acide 2-propyl-4-(4-(2-pyridyl)piperazin-1-ylméthyl)-1-(2'-(1H-tétrazol-5-yl)biphényl-4-ylméthyl)imidazole-5-carboxylique,

XR-510 : 1-((2'((iso-Pentyloxycarbonylamino)sulfonyl)-3-fluoro-(1,1'-biphényl)-4-yl)méthyl)-5- [2-(N-butyryl-N-pyridin-3-ylamino)propionyl]-4-éthyl-2-propyl-1H-imidazole, sel de potassium,

YM-358 : 2,7-Diéthyl-5-[[2'-(1H-tétrazol-5-yl)[1,1'-biphényl]-4-yl]méthyl-5H-pyrazolo[1,5-b][1,2,4]-triazole,

YM-31 472 : Ester 1-(éthoxycarbonyloxy)éthylique de l'acide 2-Butyl-4-(3,N-diméthyl-2-buténamido)- 1-(2'-(1H-tétrazol-5-yl)biphényl-4-ylméthyl)imidazole-5-carboxylique,

l'irbesartan étant particulièrement préféré, soit tel quel, soit sous forme polymorphe, soit sous forme d'un de ses sels ou solvates.

Selon la présente invention, on a maintenant trouvé que l'utilisation d'un antagoniste de l'angiotensine II et d'un dérivé du benzofurane à activité antiarythmique se traduit par un synergie des effets dans le traitement des affections cardiovasculaires notamment dans le traitement de la défaillance cardiaque aigüe ou chronique.

Plus particulièrement, l'utilisation d'un antagoniste de l'angiotensine II et de l'amiodarone ou de la dronédarone et de ses sels ou d'un de leurs métabolites actifs se traduit par une synergie des effets dans le traitement de la défaillance cardiaque aigüe ou chronique permettant ainsi d'augmenter la survie des patients et de diminuer la mortalité.

Ainsi, la présente invention a pour objet l'utilisation d'un antagoniste de l'angiotensine II et d'un dérivé du benzofurane à activité antiarythmique pour la préparation d'une composition pharmaceutique utile dans le traitement des affections cardiovasculaires, en particulier dans le traitement de la défaillance cardiaque aigüe ou chronique. Plus particulièrement, la présente invention a pour objet l'utilisation d'un antagoniste de l'angiotensine II et de l'amiodarone ou de la dronédarone et de ses sels ou d'un de leurs métabolites actifs tels que mentionnés plus haut pour la préparation d'une composition pharmaceutique utile notamment dans le traitement de la défaillance cardiaque aigüe ou chronique.

Pour ladite utilisation, un antagoniste de l'angiotensine II préféré est l'irbesartan.

Selon la présente invention, l'association avec un inhibiteur de l'angiotensine II peut permettre de diminuer la dose de dérivé de benzofurane à activité antiarythmique administré. Ainsi, avantageusement, les effets indésirables de l'amiodarone pourront être notablement diminués.

Lors de l'administration au patient de l'association selon l'invention (antagoniste de l'angiotensine II et dérivé du benzofurane) on pourra administrer simultanément tout autre médicament utile, tel que par exemple un digitalique ou un diurétique.

La présente invention a également pour objet une nouvelle composition pharmaceutique caractérisée en ce qu'elle comprend un dérivé de benzofurane à activité antiarythmique et un composé inhibiteur de l'angiotensine II avec un excipient pharmaceutique. En particulier l'invention a pour objet une nouvelle composition pour le traitement des affections cardiovasculaires, notamment la défaillance cardiaque, caractérisé en ce qu'elle comprend un dérivé de benzofurane à activité antiarythmique et un composé inhibiteur de l'angiotensine II avec un excipient pharmaceutique.

Plus particulièrement, une telle composition comprend :

- l'amiodarone et un inhibiteur de l'angiotensine II, préférentiellement l'irbesartan, avec un excipient pharmaceutique,

         ou bien :

- la dronédarone ou un de ses sels et un inhibiteur de l'angiotensine II, préférentiellement l'irbesartan, avec un excipient pharmaceutique,

         ou bien :

- la N-deséthylamiodarone et un inhibiteur de l'angiotensine II, préférentiellement l'irbesartan, avec un excipient pharmaceutique,

- la N-butyl-3-[4-((2-butyl-5-méthylsulfonamido)benzofuran-3-yl-carbonyl) phénoxy]propylamine et un inhibiteur de l'angiotensine II, préférentiellement l'irbesartan, avec un excipient pharmaceutique.

Préférentiellement, il s'agit d'une composition comprenant :

     10 mg à 600 mg de dérivé de benzofurane à activité antiarythmique

     et 1 mg à 500 mg de composé inhibiteur de l'angiotensine II.

Plus particulièrement, cette composition comprend :

     50 mg à 400 mg d'amiodarone et 20 mg à 200 mg d'irbesartan

         ou bien :

     50 mg à 400 mg de N,N-dibutyl-3-[4-((2-butyl-5-méthylsulfonamido)benzofuran-3-yl-carbonyl)phénoxy]propylamine et 20 mg à 200 mg d'irbesartan.

La composition pharmaceutique selon l'invention pourra être administrée 1 à 5 fois par jour ; préférentiellement 1 à 2 fois par jour, mieux 1 fois par jour.

Une telle composition est obtenue par mélange avec les excipients galéniquement convenables pour obtenir une composition pour administration par voie orale, parentérale, nasale, inhalée, topique, transcutanée, rectale. L'administration par voie orale étant préférée. Ainsi on peut préparer des comprimés, des capsules, des granules, des poudres, des solutions ou des suspensions.

Selon un autre aspect de l'invention, l'antagoniste de l'angiotensine II et le dérivé de benzofurane à activité anti-arythmique mentionnés ci-dessus peuvent être administrés de manière séquentielle pour le traitement des affections cardiovasculaires notamment de la défaillance cardiaque.

L'invention concerne donc également une trousse pour le traitement des affections cardiovasculaires, notamment pour le traitement de la défaillance cardiaque aigüe ou chronique par administration séquentielle d'un antagoniste de l'angiotensine II et d'un dérivé de benzofurane à activité anti-arythmique, caractérisée en ce que ledit antagoniste de l'angiotensine II et ledit dérivé de benzofurane sont dans des conditionnements distincts. Plus particulièrement, ladite trousse contient un antagoniste de l'angiotensine II, en particulier l'irbesartan, et l'amiodarone, ou la dronédarone ou un de ses sels, ou un de leurs métabolites actifs tels que mentionnés plus haut.

Les exemples suivants illustrent l'invention.

EXEMPLE 1 Comprimé

| | |
|---|---|
| Amiodarone chlorhydrate | 150 mg |
| Irbesartan | 25 mg |
| Lactose | 48,5 mg |
| Amidon de maïs | 44 mg |
| Talc | 25 mg |
| Polyvinylpyrolidone | 9 mg |
| Silice colloïdale anhydre | 0,5 mg |
| Stéarate de magnésium | 3,0 mg |
| Eau purifié Q/S | 300 mg |

EXEMPLE 2 Comprimé

| | |
|---|---|
| Amiodarone chlorhydrate | 200 mg |
| Irbesartan | 50 mg |
| Lactose | 72,5 mg |
| Amidon de maïs modifié | 48 mg |
| Talc | 25 mg |
| Silice colloïdale anhydre | 0,5 mg |
| Stéarate de magnésium | 4,0 mg |
| | 400 mg |

EXEMPLE 3 Solution injectable i.v

| | |
|---|---|
| Amiodarone chlorhydrate | 200 mg |
| Irbesartan | 25 mg |
| Polysorbate 80 | 500 mg |
| Alcool benzylique | 100 mg |
| Eau pour préparation injectable Q/S | 5 ml |

EXEMPLE 4 Comprimé

| | |
|---|---|
| Dronédarone | 150 mg |
| Irbesartan | 25 mg |
| Lactose | 47,5 mg |
| Amidon de maïs | 40 mg |
| Talc | 25 mg |
| Hydroxypropylméthylcellulose | 9 mg |
| Silice colloïdale anhydre | 0,5 mg |
| Stéarate de magnésium | 3,0 mg |
| Eau purifiée Q/S | |
| | 300 mg |

EXEMPLE 5 Comprimé

| Amiodarone chlorhydrate | 150 mg |
| Inhibiteur de l'angiotensine II | 50 mg |
| Lactose | 52,5 mg |
| Amidon de maïs | 20 mg |
| Talc | 15 mg |
| Polyvinylpyrolidone | 9 mg |
| Silice colloïdale anhydre | 0,5 mg |
| Stéarate de magnésium | 3,0 mg |
| Eau purifiée Q/S | |
| | 300 mg |

EXEMPLE 6 Comprimé

| Amiodarone chlorhydrate | 100 mg |
| Inhibiteur de l'angiotensine II | 25 mg |
| Lactose | 128,5 mg |
| Amidon de maïs modifié | 50 mg |
| Talc | 25 mg |
| Silice colloïdale anhydre | 0,5 mg |
| Stéarate de magnésium | 1,0 mg |
| Eau purifiée - Q/S | |
| | 400 mg |

**Revendications**

1. Utilisation d'un antagoniste de l'angiotensine II et d'un dérivé du benzofurane à activité antiarythmique pour la préparation d'une composition pharmaceutique utile dans le traitement des affections cardiovasculaires.

2. Utilisation selon la revendication 1 pour la préparation d'une composition pharmaceutique utile dans le traitement de la défaillance cardiaque aigüe ou chronique.

3. Utilisation selon l'une quelconque des revendications 1 ou 2 d'un antagoniste de l'angiotensine II et de l'amiodarone ou de la N,N-dibutyl-3-[4-((2-butyl-5-méthylsulfonamido)benzofuran-3-yl-carbonyl) phénoxy]propylamine et de ses sels ou de la N-déséthylamiodarone ou de la N-butyl-3-[4-((2-butyl-5-méthylsulfonamido)benzofuran-3-yl-carbonyl) phénoxy]propylamine.

4. Utilisation selon l'une quelconque des revendications 1, 2 ou 3 dans laquelle l'antagoniste de l'angiotensine II est l'irbesartan.

5. Composition pharmaceutique caractérisée en ce qu'elle comprend un dérivé de benzofurane à activité antiarythmique et un composé inhibiteur de l'angiotensine II avec un excipient pharmaceutique.

6. Composition pharmaceutique selon la revendication 5 comprenant l'amiodarone et un inhibiteur de l'angiotensine II, avec un excipient pharmaceutique.

7. Composition pharmaceutique selon la revendication 5 comprenant la N,N-dibutyl-3-[4-((2-butyl-5-méthylsulfonamido)benzofuran-3-yl-carbonyl) phénoxy]propylamine ou un de ses sels et un inhibiteur de l'angiotensine II, avec un excipient pharmaceutique.

8. Composition pharmaceutique selon la revendication 5 comprenant la N-déséthylamiodarone et un inhibiteur de l'angiotensine II, avec un excipient pharmaceutique.

9. Composition pharmaceutique selon la revendication 5 comprenant la N-butyl-3-[4-((2-butyl-5-méthylsulfonamido)benzofuran-3-yl-carbonyl)phénoxy] propylamine et un inhibiteur de l'angiotensine II, avec un excipient pharmaceutique.

10. Composition pharmaceutique selon l'une quelconque des revendications 6, 7, 8 ou 9 dans laquelle l'inhibiteur de l'angiotensine II est l'irbesartan.

11. Composition pharmaceutique selon la revendication 5 comprenant 10 mg à 600 mg de dérivé de benzofurane à activité antiarythmique et 1 mg à 600 mg de composé inhibiteur de l'angiotensine II.

12. Composition pharmaceutique selon l'une quelconque des revendications 5, 6 ou 10 comprenant 50 mg à 400 mg d'amiodarone et 20 mg à 200 mg d'irbesartan.

13. Composition pharmaceutique selon l'une quelconque des revendications 5, 6 ou 10 comprenant 50 mg à 400 mg de la N,N-dibutyl-3-[4-((2-butyl-5-méthylsulfonamido)benzofuran-3-yl-carbonyl)phénoxy]propylamine et 20 mg à 200 mg d'irbesartan.

14. Composition pharmaceutique selon l'une quelconque des revendications 5 à 13 pour administration orale.

15. Composition pharmaceutique selon l'une quelconque des revendications 5 à 14 pour le traitement des affections cardiovasculaires.

16. Composition pharmaceutique selon l'une quelconque des revendications 5 à 14 pour le traitement de la défaillance cardiaque aigue ou chronique.

**17.** Trousse pour le traitement des affections cardiovasculaires, notamment pour le traitement de la défaillance cardiaque aigüe ou chronique par administration séquentielle d'un antagoniste de l'angiotensine II et d'un dérivé de benzofurane à activité anti-arythmique, caractérisée en ce que ledit antagoniste de l'angiotensine II et ledit dérivé de benzofurane sont dans des conditionnements distincts.

**18.** Trousse selon la revendication 17, caractérisée en ce que l'antagoniste de l'angiotensine II est l'irbesartan.

**19.** Trousse selon la revendication 17 ou 18, caractérisée en ce que le dérivé de benzofurane à activité anti-arythmique est choisi parmi l'amiodarone, la N,N-dibutyl-3-(4-((2-butyl-5-méthylsulfonamido)benzofuran-3-yl-carbonyl)phénoxy]propylamine ou un de ses sels, la N-déséthylamiodarone, et la N-butyl-3-[4-((2-butyl-5-méthylsulfonamido)benzofuran-3-yl-carbonyl)phénoxy]propylamine.